# EUROPÄISCHE PATENTSCHRIFT

(11) **EP 1 123 392 B1**
(45) Veröffentlichungstag und Bekanntmachung des Hinweises auf die Patenterteilung: **13.12.2006**
(21) Anmeldenummer: 99947439.8
(22) Anmeldetag: 30.09.1999
(51) Int. Cl.: C07K 14/475, C07K 16/22, C12Q 1/68, A61K 39/395, A61K 38/18

(54) **DAS GEN PRV-1 UND DESSEN VERWENDUNG**
PRV-1 GENE AND THE USE THEREOF
LE GENE PRV-1 ET SON UTILISATION

(30) Priorität: 23.10.1998 DE 19849044
(43) Veröffentlichungstag der Anmeldung: 16.08.2001
(73) Patentinhaber: Universitätsklinikum Freiburg, 79106 Freiburg (DE)
(72) Erfinder: PAHL, Heike, D-79112 Freiburg (DE)
(74) Vertreter: Keller, Günter
(86) Internationale Anmeldenummer: PCT/EP1999/007238
(87) Internationale Veröffentlichungsnummer: WO 2000/024886

(56) Entgegenhaltungen:
- WO-A-98/50552
- DATABASE EMBL - EMEST7 [Online] Entry/Acc.no. AI205247, 19. Oktober 1998 (1998-10-19) HILLIER, L. ET AL.: "ao85e03.x1 Schiller meningioma Homo sapeins cDNA clone IMAGE:1952668 3', mRNA sequence." XP002129279
- TEMERINAC, S. ET AL.: "Cloning of PRV-1, a novel gene overexpressed in polycythemia vera." ANNALS OF HEAMATOLOGY, Bd. 77, Nr. Suppl.II, 25. Oktober 1998 (1998-10-25), Seiten S110-Abstr.434, XP000867769

### SEQUENZPROTOKOLL

<110> Universitätsklinikum Freiburg
<120> Das Gen PRV-1 und dessen Verwendung
<130> E980930
<140> PCT/EP99/07238
   <141> 1999-09-30
<150> 198 49 044.5
   <151> 1998-10-23
<160> 9
<170> PADAT Sequenzmodul, Version 1.0
<210> 1
   <211> 1600
   <212> DNA
   <213> homo sapiens
<220>
   <223>
<400> 1
   AAAAGCAGAA AGAGATTACC AGCCACAGAC GGGTCATGAG CGCGGTATTA CTGCTGGCCC 60
   TCCTGGGGTT CATCCTCCCA CTGCCAGGAG TGCAGGCGCT GCTCTGCCAG TTTGGGACAG 120
   TTCAGCATGT GTGGAAGGTG TCCGACCTGC CCCGGCAATG GACCCCTAAG AACACCAGCT 180
   GCGACAGCGG CTTGGGGTGC CAGGACACGT TGATGCTCAT TGAGAGCGGA CCCCAAGTGA 240
   GCCTGGTGCT CTCCAAGGGC TGCACGGAGG CCAAGGACCA GGAGCCCCGC GTCACTGAGC 300
   ACCGGATGGG CCCCGGCCTC TCCCTGATCT CCTACACCTT CGTGTGCCGC CAGGAGGACT 360
   TCTGCAACAA CCTCGTTAAC TCCCTCCCGC TTTGGGCCCC ACAGCCCCCA GCAGACCCAG 420
   GATCCTTGAG GTGCCCAGTC TGCTTGTCTA TGGAAGGCTG TCTGGAGGGG ACAACAGAAG 480
   AGATCTGCCC CAAGGGGACC ACACACTGTT ATGATGGCCT CCTCAGGCTC AGGGGAGGAG 540
   GCATCTTCTC CAATCTGAGA GTCCAGGGAT GCATGCCCCA GCCAGGTTGC AACCTGCTCA 600
   ATGGGACACA GGAAATTGGG CCCGTGGGTA TGACTGAGAA CTGCAATAGG AAAGATTTTC 660
   TGACCTGTCA TCGGGGGACC ACCATTATGA CACACGGAAA CTTGGCTCAA GAACCCACTG 720
   ATTGGACCAC ATCGAATACC GAGATGTGCG AGGTGGGGCA GGTGTGTCAG GAGACGCTGC 780
   TGCTCATAGA TGTAGGACTC ACATCAACCC TGGTGGGGAC AAAAGGCTGC AGCACTGTTG 840
   GGGCTCAAAA TTCCCAGAAG ACCACCATCC ACTCAGCCCC TCCTGGGGTG CTTGTGGCCT 900
   CCTATACCCA CTTCTGCTCC TCGGACCTGT GCAATAGTGC CAGCAGCAGC AGCGTTCTGC 960
   TGAACTCCCT CCCTCCTCAA GCTGCCCCTG TCCCAGGAGA CCGGCAGTGT CCTACCTGTG 1020
   TGCAGCCCCT TGGAACCTGT TCAAGTGGCT CCCCCCGAAT GACCTGCCCC AGGGGCGCCA 1080
   CTCATTGTTA TGATGGGTAC ATTCATCTCT CAGGAGGTGG GCTGTCCACC AAAATGAGCA 1140
   TTCAGGGCTG CGTGGCCCAA CCTTCCAGCT TCTTGTTGAA CCACACCAGA CAAATCGGGA 1200
   TCTTCTCTGC GCGTGAGAAG CGTGATGTGC AGCCTCCTGC CTCTCAGCAT GAGGGAGGTG 1260
   GGGCTGAGGG CCTGGAGTCT CTCACTTGGG GGGTGGGGCT GGCACTGGCC CCAGCGCTGT 1320
   GGTGGGGAGT GGTTTGCCCT TCCTGCTAAC TCTATTACCC CCACGATTCT TCACCGCTGC 1380
   TGACCACCCA CACTCAACCT CCCTCTGACC TCATAACCTA ATGGCCTTGG ACACCAGATT 1440
   CTTTCCCATT CTGTCCATGA ATCATCTTCC CCACACACAA TCATTCATAT CTACTCACCT 1500
   AACAGCAACA CTGGGGAGAG CCTGGAGCAT CCGGACTTGC CCTATGGGAG AGGGGACGCT 1560
   GGAGGAGTGG CTGCATGTAT CTGATAATAC AGACCCTGTC 1600
<210> 2
   <211> 437
   <212> PRT
   <213> homo sapiens
<400> 2
   Met Ser Ala Val Leu Leu Leu Ala Leu Leu Gly Phe Ile Leu Pro Leu
   1               5                   10                  15
   Pro Gly Val Gln Ala Leu Leu Cys Gln Phe Gly Thr Val Gln His Val
               20                  25                  30
   Trp Lys Val Ser Asp Leu Pro Arg Gln Trp Thr Pro Lys Asn Thr Ser
           35                  40                  45
   Cys Asp Ser Gly Leu Gly Cys Gln Asp Thr Leu Met Leu Ile Glu Ser
       50                  55                  60
   Gly Pro Gln Val Ser Leu Val Leu Ser Lys Gly Cys Thr Glu Ala Lys
   65                  70                  75                  80
   Asp Gln Glu Pro Arg Val Thr Glu His Arg Met Gly Pro Gly Leu Ser
                   85                  90                  95
   Leu Ile Ser Tyr Thr Phe Val Cys Arg Gln Glu Asp Phe Cys Asn Asn
               100                 105                 110
   Leu Val Asn Ser Leu Pro Leu Trp Ala Pro Gln Pro Pro Ala Asp Pro
           115                 120                 125
   Gly Ser Leu Arg Cys Pro Val Cys Leu Ser Met Glu Gly Cys Leu Glu
       130                 135                 140
   Gly Thr Thr Glu Glu Ile Cys Pro Lys Gly Thr Thr His Cys Tyr Asp
   145                 150                 155                 160
   Gly Leu Leu Arg Leu Arg Gly Gly Gly Ile Phe Ser Asn Leu Arg Val
                   165                 170                 175
   Gln Gly Cys Met Pro Gln Pro Gly Cys Asn Leu Leu Asn Gly Thr Gln
               180                 185                 190
   Glu Ile Gly Pro Val Gly Met Thr Glu Asn Cys Asn Arg Lys Asp Phe
           195                 200                 205
   Leu Thr Cys His Arg Gly Thr Thr Ile Met Thr His Gly Asn Leu Ala
       210                 215                 220
   Gln Glu Pro Thr Asp Trp Thr Thr Ser Asn Thr Glu Met Cys Glu Val
   225                 230                 235                 240
   Gly Gln Val Cys Gln Glu Thr Leu Leu Leu Ile Asp Val Gly Leu Thr
                   245                 250                 255
   Ser Thr Leu Val Gly Thr Lys Gly Cys Ser Thr Val Gly Ala Gln Asn
               260                 265                 270
   Ser Gln Lys Thr Thr Ile His Ser Ala Pro Pro Gly Val Leu Val Ala
           275                 280                 285
   Ser Tyr Thr His Phe Cys Ser Ser Asp Leu Cys Asn Ser Ala Ser Ser
       290                 295                 300
   Ser Ser Val Leu Leu Asn Ser Leu Pro Pro Gln Ala Ala Pro Val Pro
   305                 310                 315                 320
   Gly Asp Arg Gln Cys Pro Thr Cys Val Gln Pro Leu Gly Thr Cys Ser
                   325                 330                 335
   Ser Gly Ser Pro Arg Met Thr Cys Pro Arg Gly Ala Thr His Cys Tyr
               340                 345                 350
   Asp Gly Tyr Ile His Leu Ser Gly Gly Gly Leu Ser Thr Lys Met Ser
           355                 360                 365
   Ile Gln Gly Cys Val Ala Gln Pro Ser Ser Phe Leu Leu Asn His Thr
   370 375 380
   Arg Gln Ile Gly Ile Phe Ser Ala Arg Glu Lys Arg Asp Val Gln Pro
   385 390 395 400
   Pro Ala Ser Gln His Glu Gly Gly Gly Ala Glu Gly Leu Glu Ser Leu
   405 410 415
   Thr Trp Gly Val Gly Leu Ala Leu Ala Pro Ala Leu Trp Trp Gly Val
   420 425 430
   Val Cys Pro Ser Cys
   435
<210> 3
   <211> 24
   <212> RNA
   <213> homo sapiens
<220>
   <223>
<400> 3
   AAAAGCAGAA AGAGATTACC AGCC 24
<210> 4
   <211> 24
   <212> RNA
   <213> homo sapiens
<220>
   <223>
<400> 4
   GGCTGGTAAT CTCTTTCTGC TTTT 24
<210> 5
   <211> 13
   <212> PRT
   <213> homo sapiens
<400> 5
   Lys Val Ser Asp Leu Pro Arg Gln Trp Thr Pro Lys Asn
   1 5 10
<210> 6
   <211> 15
   <212> PRT
   <213> homo sapiens
<400> 6
   Ser Ala Arg Glu Lys Arg Asp Val Gln Pro Pro Ala Ser Gln His
   1 5 10 15
<210> 7
   <211> 27
   <212> DNA
   <213> homo sapiens
<220>
   <223>
<400> 7
   ATTAGGTTAT GAGGTCAGAG GGAGGTT 27
<210> 8
   <211> 28
   <212> DNA
   <213> homo sapiens
<220>
   <223>
<400> 8
   GCAGAAAGAG ATTACCAGCC ACAGACGG 28
<210> 9
   <211> 28
   <212> DNA
   <213> homo sapiens
<220>
   <223>
<400> 9
   GAATCGTGGG GGTAATAGAG TTAGCAGG 28

## Beschreibung

Die Erfindung offenbart eine Nucleotidsequenz, die das PRV-1-Gen codiert, rekombinante DNA, die diese Nucleotidsequenz enthält, Vektoren, die die rekombinante DNA enthalten und damit transformierte Zellen, sowie ein PRV-1-Polypeptid, Antikörper gegen dieses Polypeptid und betrifft ein Verfahren zum Nachweis des PRV-1-Polypeptids.

Die Polycythaemia rubra vera, auch als Polycythaemia vera oder P. vera bezeichnet, ist eine maligne hämatologische Erkrankung, bei der eine vermehrte Bildung erythroider, granulozytärer und megakaryozcytärer Zellen vorliegt. Die Erkrankung ist klonalen Ursprungs und entsteht durch Mutation einer einzigen hämatopoietischen Vorläuferzelle. Die Inzidenz der P. vera beträgt 4 bis 6 pro Million Einwohner in Deutschland. Unbehandelt führt die Krankheit innerhalb von 18 Monaten zum Tod. Eine Behandlung durch Aderlässe oder Chemotherapie verlängert die durchschnittliche Überlebensdauer auf über 13 Jahre.

Die Diagnose der P. vera erfolgt über klinische Kriterien. Zum klinischen Bild gehören Kopfschmerzen, Puritus, Splenomegalie bei zwei Drittel der Patienten, Blutungen oder Thrombosen, Bluthochdruck bei einem Drittel der Patienten, Gicht, ausgelöst durch gesteigerte Harnsäureproduktion und in manchen Fällen septische Ulcera. Der wichtigste Laborbefund ist eine Erhöhung der Werte für Hämoglobin, Hämatokrit, Erythrozytenzahl und Erythrozytengesamtvolumen sowie eine neutrophile Granulozytose oder Thrombozytose in vielen Fällen. Da einerseits die meisten Kriterien eher diffus sind und andererseits nicht alle Patienten diese Kriterien erfüllen, ist es häufig schwierig, die P. vera von anderen myeloproliferativen Erkrankungen, wie chronischer myeloischer Leukämie, oder essentieller Thrombozytämie, abzugrenzen und damit die Diagnose zu sichern. Die molekulare Ursache der P. vera ist bisher vollkommen unbekannt. Da aber die P. vera, wenn sie nicht behandelt wird, einen schweren Verlauf nimmt, ist eine genaue Diagnose wichtig.

Eine Aufgabe der Erfindung war es daher, die molekulare Ursache der Polycythaemia rubra vera aufzufinden und eine Möglichkeit zu ihrer Diagnose zu schaffen.

Diese Aufgabe wurde gelöst, indem ein Gen isoliert wurde, das spezifisch bei P. vera und nicht bei gesunden Kontrollen exprimiert wird. Dieses Gen wird als PRV-1-Gen (Polycythaemia rubra vera) bezeichnet.

Eine ähnliche Nucleotidsequenz wird in der internationalen Anmeldung WO 98/50552 offenbart.
Der Schutzbereich des vorliegenden Patentes wird durch die Patentansprüche bestimmt.

Ein Gegenstand der Erfindung ist daher ein Polynucleotid, das für das PRV-1-Gen codiert und im wesentlichen die Sequenz ID Nr. 1 umfaßt. Die Polynucleotide der vorliegenden Erfindung können einzel- oder doppelsträngige DNA oder RNA sein. Falls es sich um RNA handelt, ist dem Fachmann klar, daß anstelle von "T"-Nucleotiden "U"-Nucleotide vorliegen. Unter "Polynucleotid" sind Nucleinsäuren mit 15 oder mehr. Nucleotiden zu verstehen.

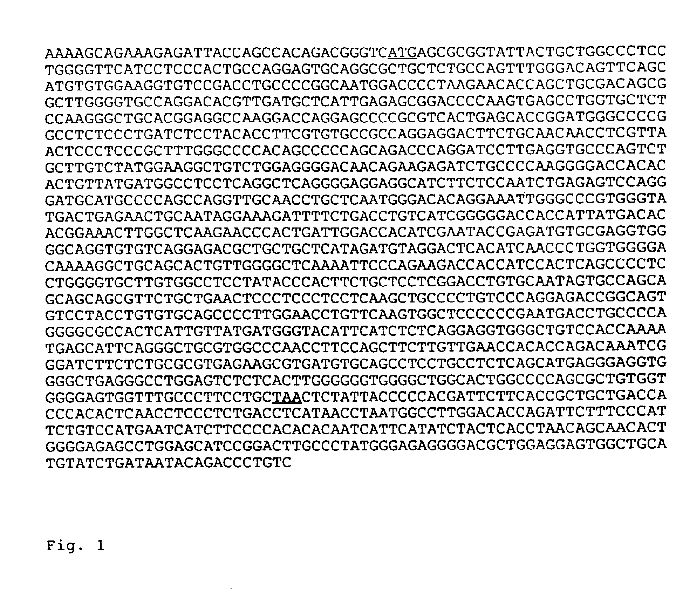

Die erfindungsgemäße Nucleotidsequenz ist in Figur 1 wiedergegeben. Verwendet werden kann ein Polynucleotid, das der Sequenz von Figur 1 entspricht, sowie ein Polynucleotid, dessen Nucleotidsequenz geringfügige Abweichungen aufweist. Unter geringfügigen Abweichungen werden im Sinne der vorliegenden Anmeldung solche Sequenzen verstanden, bei denen einige wenige, vorzugsweise nicht mehr als 50 und besonders bevorzugt nicht mehr als 25 Nucleotide ausgetauscht sein können, wobei jedoch die Funktion des durch die Nucleotidsequenz kodierten Gens nicht berührt wird. Dem Fachmann ist bekannt, daß ein für eine Aminosäure kodierendes Basentriplett durch ein anderes Triplett ersetzt werden kann, das für dieselbe Aminosäure kodiert. Darüber hinaus können weniger wichtige Bereich geringfügig deletiert und/oder mutiert sein. In einer besonderen Ausführungsform umfaßt das Polynucleotid die Nucleotide 36 bis 1346 der Sequenz Nr. 1, also den kodierenden Bereich des PRV-1-Gens. Eine weitere Ausführungsform umfaßt die Nucleotide 36 bis 1262 von Sequenz Nr. 1. Dieser Bereich kodiert vermutlich für den aktiven Bereich des PRV-1-Polypeptids. Das Polynucleotid kann schließlich auch die Nucleotide 39 bis 1346 oder 39 bis 1262 von Sequenz Nr. 1 umfassen, so daß das Codon, das für das Start-Methionin kodiert, nicht enthalten ist. Eine bevorzugte Ausführungsform ist ein Polynucleotid, das die Nucleotide 99-1346 oder 99 bis 1262 von Sequenz Nr. 1 umfaßt. Es sind damit die Codons am 5'-Ende, die für das Signalpeptid des PRV-1-Polypeptids kodieren, nicht enthalten.

Das Polynucleotid kann auch ein Fragment des PRV-1-Gens sein. Das Fragment weist in der Regel mehr als 100 Nucleotide auf, bevorzugt aber mehr als 300 Nucleotide. Die Fragmente können auch als Primer oder als Sonden insbesondere für die PCR eingesetzt werden, in diesem Fall können die Fragmente dem Zweck entsprechend verkürzt sein. Üblicherweise haben Primer eine Länge zwischen 10 und 30 Nucleotiden und Sonden eine Länge zwischen 15 und 50 Nucleotiden.

Das PRV-1-Gen ist ein körpereigenes Gen, das jedoch bei gesunden Personen nur auf wenige Organe beschränkt exprimiert wird. Normalerweise wird es im wesentlichen in den blutbildenden Organen, d.h. im Knochenmark und fötaler Leber, und schwach in der Milz exprimiert, nicht jedoch in Herz, Muskel, Pankreas oder Niere. Bei Patienten, die unter P. vera leiden, wird dieses Gen, vor allem in den hämatopoietischen Zellen, sehr stark überexprimiert.

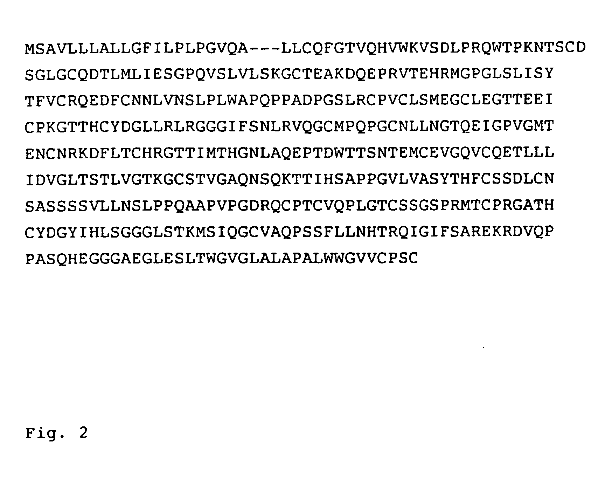

Das PRV-1-Gen kodiert für ein Protein, das die in Figur 2 gezeigte Proteinsequenz aufweist. Das Signalpeptid, das in der Proteinsequenz sämtlicher Oberflächenmoleküle enthalten ist und bei der Prozessierung des Proteins üblicherweise entfernt wird, ist durch einen Bindestrich abgetrennt. Das Protein hat die Sequenz ID Nr. 2. Ein weiterer Aspekt der Erfindung ist also ein im wesentlichen reines Polypeptid der Sequenz Nr. 2 oder ein Polypeptid der Sequenz Nr. 2, bei dem das Signalpeptid nicht vorhanden ist (Aminosäuren 22 bis 437 von Sequenz Nr. 2). Weitere Ausführungsformen umfassen die Aminosäuren 1 bis 409 oder 22 bis 409 der Sequenz Nr. 2 (vermutlich aktiver Bereich des Proteins).

Das Polypeptid ist im Hinblick auf die biologische Aktivität vorzugsweise glycosyliert, am bevorzugtesten ist es N-glycosyliert. Es kann dann an wenigstens einer der Aminosäuren Asn-46, Asn-189 und Asn-382 des PRV-1-Polypeptids glycosyliert sein (Die Aminosäurenzahlen beziehen sich auf die Sequenz Nr. 2). Offenbart werden auch Fragmente der erfindungsgemäßen Polypeptide, die N-glycosyliert sind. Die Fragmente sind wenigstens 50 Aminosäuren lang, bevorzugt wenigstens 100 Aminosäuren, am bevorzugtesten wenigstens 150 Aminosäuren. In einer anderen Ausführungsform kann das Polypeptid O-glycosylisert sein.

Dem Fachmann ist klar, daß bestimmte Aminosäuren gegen andere ausgetauscht sein können ohne die biologische Aktivität des Proteins zu beeinträchtigen. Solche abgewandelten Formen der erfindungsgemäßen Polypeptide sind auch verwendbar.
Bei den Aminosäureaustauschen handelt es sich um solche, die die biologische Aktivität des Proteins nicht negativ beeinträchtigen. Für die Auswahl der Austausche kann der Fachmann auf allgemein bekannte Regeln zurückgreifen.

Das PRV-1-Polypeptid kann herstellungsbedingt beispielsweise einen Glycosylphosphatidylinositol-Anker aufweisen. Dieser ist dann an den Aminosäuren gebunden, die den Aminosäuren 407 bis 409 der Sequenz ID Nr. 2 entsprechen. Ein GPI-Anker dient dazu, ein Protein mittels eines Lipids auf der Außenseite der Zellmembran zu verankern. Aus bislang nicht endgültig geklärten Gründen beobachtet man aber häufig, daß GPI-gelinkte Proteine auch ins Medium abgegeben werden. Man spricht von einem sogenannten "shedding". Ob dies ein spezifischer Prozeß ist, das heißt solche Proteine in einer kontrollierten Weise von Enzymen aus der Membran abgespalten werden, oder ob es ein unspezifischer Verlust des Ankers ist, ist bislang nicht geklärt. Es ist also sehr wahrscheinlich, daß PRV-1 sowohl an der Zellmembran als auch extrazellulär zu finden ist. Für die Wirkung als Wachstumsfaktor ist wahrscheinlich die sezernierte, nicht Membran-gebundene Form wichtiger, da sie als Wachstumsfaktor diffundieren und andere Zellen erreichen kann.

Dem Fachmann ist klar, daß er durch Manipulation dieser Aminosäuren die Membranständigkeit des Proteins beeinflussen kann. Dies betrifft vor allem die Herstellung bestimmter DNA-Konstrukte, die zur Expression des PRV-1-Polypeptids oder von Fragmenten davon bestimmt sind. Die Codons, die für diese Aminosäuren kodieren, können mutiert oder deletiert sein.

Das Gen codiert für einen Oberflächenrezeptor der uPAR/Ly6-Familie. Diese Rezeptorenfamilie kann mitogene Signale, d.h.

Signale, die die Zellteilung anregen, übertragen. Es wird daher angenommen, daß die Überexpression des PRV-1-Gens, unter anderem auf den Granulozyten von P. vera-Patienten, zu einer Hyperproliferation dieser Zellen beiträgt.

Es wurde gefunden, daß weder bei gesunden Personen noch bei Patienten mit anderen myeloproliferativen Erkrankungen, z.B. mit chronischer myeloischer Leukämie, akuter myeloischer Leukämie und essentieller Thrombozytämie, PRV-1 auf Granulozyten exprimiert wird.

Um das von dem PRV-1-Gen codierte Polypeptid für Analysen und Nachweisverfahren einsetzen zu können, wird es geeigneter Weise aus rekombinanter DNA erzeugt, wobei die rekombinante DNA bevorzugt die Nucleotidsequenz ID Nr. 1 oder wenigstens den kodierenden Bereich des PRV-1-Gens, also die Nucleotide 36 bis 1346 von Sequenz ID Nr. 1, zumindest aber die Nucleotide 39 bis 1262, funktionell verbunden mit einem Promotor, umfaßt. Die rekombinante DNA kann jedoch auch nur ein Fragment der Sequenz Nr. 1 umfassen.

Zur Herstellung eingesetzt werden können ein Vektor, der die rekombinante DNA für das PRV-1-Polypeptid oder ein Fragment davon enthält, sowie eine mit diesem Vektor transfizierte oder transformierte Wirtszelle. Die Wirtszellen können prokaryontisch sein, beispielsweise Bakterien wie E. coli. Dabei werden jedoch nicht-glycosylierte Polypeptide exprimiert. Bevorzugt sind daher eukaryontische Wirtszellen, die das exprimierte Protein posttranslational glycosylieren und anderweitig modifizieren können. Beispiele für eukaryontische Wirtszellen sind Insektenzellen wie Sf9-Zellen zur Expression nach Infektion mit rekombinanten Baculoviren, Säugerzellen wie COS-Zellen, CHO-Zellen, HeLa-Zellen. Diese Beispiele sind nicht erschöpfend. Auch Hefezellen sind als Wirtszellen möglich. Dem Fachmann ist klar, daß je nach Wirtszelle das Glykosylierungsmuster unterschiedlich sein kann. Damit kann auch die biologische Aktivität des Expressionsprodukts variieren. Besonders bevorzugt sind Wirtszellen, die das Expressionsprodukt derart glycosylieren, daß die biologische Aktivität des Proteins erhalten ist.

Das aus Granulozyten gewonnene oder rekombinant erzeugte PRV-1-Polypeptid kann sowohl zur Diagnose von Polycythämia vera als auch zur Behandlung der Krankheit eingesetzt werden.

Eine Möglichkeit der Therapie besteht in der sogenannten "Antisense-Therapie". Bei diesem Verfahren wird ein "Antisense"-RNA-Molekül, also eine RNA, die komplementär ist zu der PRV-RNA, eingesetzt. Da die PRV-1-RNA an ihrem Anfang die Sequenz 5'-AAAAGCAGAAAGAGATTACCAGCC-3' (Seq. ID-No. 3) hat, würde die erforderliche Antisense-RNA gegen diese Sequenz die folgende Nucleotidsequenz aufweisen: 5'-GGCTGGTAATCTCTTTCTGCTTTT-3' (Seq. ID-No. 4). Diese Antisense-RNA wird in einen Vektor eingebaut und in P. vera-Zellen eingebracht. Das Einbringen dieser RNA erfolgt beispielsweise über Transfektion, wobei der für die Transfektion verwendete Vektor vorzugsweise so gestaltet ist, daß er spezifisch in die P. vera-Zellen eingebracht wird. Die Expression der Antisense-RNA bewirkt, daß die PRV-1-mRNA nicht mehr zu einem Polypeptid translatiert werden kann. In derart behandelten Zellen entsteht dann kein PRV-1-Protein.

Ein weiterer Gegenstand der Erfindung ist daher ein Verfahren zum Nachweis von P. vera, das dadurch gekennzeichnet ist, daß man das PRV-1-Polypeptid oder ein Epitop davon nachweist und das Ausmaß der Expression bestimmt.

Eine Überexpression dieses Rezeptors auf reifen Zellen außerhalb des Knochenmarks, z.B. auf Granulozyten, ist ein starker Hinweis auf das Vorliegen der Erkrankung P. vera. Der Nachweis erfolgt geeigneterweise mit einem Immunoassay unter Verwendung von Antikörpern, die gegen den PRV-1-Rezeptor gerichtet sind. Als Testverfahren eignen sich die bekannten Varianten von Immunoassays, bei denen für das PRV-1-Polypeptid spezifische Antikörper eingesetzt werden zusammen mit weiteren markierten Antikörpern, die immobilisiert oder in Lösung sein können. Die Markierung kann in an sich bekannter Weise erfolgen, z.B. mit radioaktiven Isotopen, durch Fluoreszenz oder Lumineszenz, mit Enzymen, durch farbbildende Reaktionen oder sonstige zur Bestimmung geeigneten Gruppen. Diese Varianten sind dem Fachmann bekannt und bedürfen hier keiner näheren Erläuterung. Erfindungsgemäß werden ELISA-Tests besonders bevorzugt.

Die zum spezifischen Nachweis des PRV-1-Rezeptors erforderlichen Antikörper können ebenfalls in an sich bekannter Weise hergestellt werden. Geeignet sind sowohl monoklonale als auch polyklonale Antikörper, wobei die Verwendung monoklonaler Antikörper bevorzugt ist.

Für die Herstellung von Antikörpern können auch aus dem Protein hergeleitete Peptide benutzt werden. Im Rahmen der vorliegenden Erfindung wurden erfolgreich die Peptide mit den Sequenzen:
a) KVSDLPRQWTPKN (Aminosäuren 34 bis 46) [Seq. ID-No. 5] und
b) SAREKRDVQPPASQH (Aminosäuren 391 bis 405) [Seq. ID-No. 6]
eingesetzt.

Die polyklonalen Antikörper werden üblicherweise erzeugt, indem ein geeigneter Wirt (Kaninchen) mit dem PRV-1-Polypeptid, gegebenenfalls gebunden an einem immunologischen Träger (Adjuvans), immunisiert und eine Immunantwort hervorgerufen wird. Monoklonale Antikörper können in an sich bekannter Weise mit der Hybridoma-Technik erzeugt werden. Die Antikörper können durch Affinitätsreinigung gereinigt werden. Herstellung und Reinigung von Antikörpern sind beispielsweise beschrieben in "Antibodies: A Laboratory Manual" von Harlow und Lane, Cold Spring Harbor Laboratory Press.

Weiterhin können derartige polyklonale oder monoklonale gegen PRV-1 gerichtete Antikörper auch zur Therapie der Krankheit verwendet werden.

In einer weiteren Ausführungsform kann der Nachweis des PRV-1-Rezeptors mit einem RT-PCR-Verfahren erfolgen. Dazu wird zunächst aus den PRV-1 überexprimierenden Zellen, in der Regel Granulozyten, RNA isoliert. Dann wird in an sich bekannter Weise mit einem RT-Primer eine reverse Transkription vorgenommen. Der RT-Primer ist bevorzugt ein Primer mit der folgenden Nucleotidsequenz (SEQ ID-No. 7)
ATTAGGTTATGAGGTCAGAGGGAGGTT.

Dadurch wird die spezifische PRV-1-RNA in DNA umgewandelt. Diese DNA wird dann in einer PCR-Reaktion in an sich bekannter Weise amplifiziert. Für die Amplifikationszyklen werden bevorzugt die folgenden beiden Primer eingesetzt.
Als Primer sense (SEQ ID-No. 8)
GCAGAAAGAGATTACCAGCCACAGACGG.
Primer antisense (SEQ ID-No. 9)
GAATCGTGGGGGTAATAGAGTTAGCAGG.

Mit der offenbarten Sequenz kann der Fachmann ohne weiteres andere ebenfalls geeignete Primer auffinden.

Da die RNA als Ausgangsmaterial für dieses Verfahren verwendet wird, ist das PCR-Signal nur in solchen Fällen positiv, in denen das PRV-1-Gen auch exprimiert wird. Wie oben ausgeführt, ist dies nur dann der Fall, wenn der Patient unter P. vera leidet. Bei gesunden Patienten erfolgt keine PRV-Expression in den Granulozyten. Die Abwesenheit eines RT-PCR-Signals deutet also daraufhin, daß keine P. vera vorliegt.

In einer weiteren Alternative kann auch ein Blotting-Verfahren, bevorzugt ein Northern Blot, zur Diagnose einer P. vera benutzt werden. Für ein derartiges Verfahren wird die RNA aus Granulozyten isoliert und dann mit einem Blotting-Verfahren, z.B. Northern Blot, auf die Expression von PRV-1 untersucht. Als Sonde kann die cDNA-Sequenz von SEQ ID Nr. 1 oder ein Abschnitt der Sequenz verwendet werden. Eine Hybridisierung tritt nur dann auf, wenn die Granulozyten von einem Patienten mit P. vera stammen, da nur dann eine Expression auf den Granulozyten vorhanden ist. Die Abwesenheit einer Hybridisierung deutet daraufhin, daß die Person, von der die Granulozyten stammen, keine P. vera hat.

Für die Northern Blot-Hybridisierung kann auch ein Fragment des Gens eingesetzt werden. Ein derartiges Fragment ist üblicherweise mehr als 100 Basen lang, vorzugsweise mehr als 300 Basen lang. Alternativ hierzu können durch Verdau des Gens mit Restriktionsendonukleasen verschiedene unterschiedliche Fragmente des Gens hergestellt werden, die als Sonden im Northern Blot verwendet werden können. Wenn die Fragmente von der cDNA herstammen, liegen sie als Doppelstränge vor, die für die Hybridisierung in die Einzelstränge aufgetrennt werden müssen. Geeignete Beispiele sind das Bam HI-PstI-Fragment von Basenpaar 420 bis Basenpaar 831 oder das PstI-PstI-Fragment von Basenpaar 831 bis Basenpaar 1900.

Der Nachweis von PRV-1-mRNA und damit der PRV-1-Expression kann auch dadurch erfolgen, daß zunächst in einer RT-PCR-Reaktion die mRNA revers transkribiert wird und die cDNA anschließend amplifiziert wird und dann die amplifizierte DNA mit einer Sonde in einem Hybridisierungsverfahren nachgewiesen wird.

Bei einer positiven Diagnose muß die Krankheit behandelt werden, da sie ansonsten in relativ kurzer Zeit zum Tod führt. Hierzu können spezifische gegen PRV-1 gerichtete Antikörper verwendet werden, an die gegebenenfalls zytotoxische Komponenten gebunden sein können.

Ein weiterer Gegenstand der Erfindung ist daher ein Arzneimittel, das neben üblichen Trägern gegen den PRV-1-Rezeptor gerichtete Antikörper enthält.

Da bei der P. vera der PRV-1-Rezeptor überexprimiert wird, kommt es bei Kontakt mit dem Anti-PRV-1-Antikörper zur Bindung von vielen Antikörpern auf der Oberfläche der befallenen Granulozyten. Die Bindung vieler Antikörper an diesen Zellen ist ein Anreiz für die immunologischen Zellen, diese Zellen zu zerstören. Auf diese Weise ist eine spezifische Eliminierung der P. vera-Zellen möglich.

Überraschenderweise wurde auch gefunden, daß das PRV-1-Polypeptid hämatopoietische Aktivität aufweist. Das PRV-1-Polypeptid ist in der Lage, bestimmte hämatopoietische Vorläuferzellen zur Bildung erythroider Kolonien anzuregen. Vor allem die N-glycosylierten Polypeptide von PRV-1 weisen diese Funktion auf. Von den erfindungsgemäßen Polypeptiden sind daher die N-glycosylierten PRV-1-Polypeptide und Fragmente davon, die die Wachstumsfaktoraktivität aufweisen, bevorzugt.

Ein weiterer Aspekt der Erfindung ist daher ein Arzneimittel, das neben einem pharmazeutisch verträglichen Träger das PRV-1-Polypeptid oder ein biologisch aktives Fragment davon enthält. Bevorzugt handelt es sich um glycosyliertes PRV-1-Polypeptid, noch bevorzugter um N-glycosyliertes PRV-1-Polypeptid oder ein biologisch aktives Fragment davon. Die Erfindung betrifft auch Arzneimittel, die wenigstens ein erfindungsgemäßes Polynucleotid enthalten.

Die vorliegende Erfindung betrifft weiterhin die Verwendung von PRV-1-Polypeptid oder einem biologisch aktiven Fragment davon als Wachstumsfaktor in vivo und ex vivo. Das PRV-1-Polypeptid oder ein biologisch aktives Fragment davon kann verwendet werden zur Behandlung sämtlicher pan-Zytopenien und pan-Zytopathien im Knochenmark und in der Zirkulation (Änderung der zellulären Bestandteile des peripheren Blutes und des Knochenmarks). Die Polypeptide der vorliegenden Erfindung können beispielsweise verwendet werden zur Behandlung von Anämien bei Nierenversagen, Chemotherapie oder Ganzkörperbestrahlung, zur Behandlung von Neutropenien und Thrombozytopenien unter Chemotherapie oder Ganzkörperbestrahlung, zur ex vivo Behandlung von peripheren oder Knochenmarks-Stammzellen zur Expansion (Vermehrung) und Retransfusion in den Patienten, und zur Behandlung von Sepsis, "systemic inflammatory response syndrome" (SIRS) oder regionaler Entzündungsreaktion. Die Polypeptide der vorliegenden Erfindung oder diese enthaltende Arzneimittel können auf verschiedenste Weise appliziert werden. Die Darreichungsformen umfassen intravenöse, intramuskuläre, subkutane, intraperitoneale, orale, transdermale und transmukosale Verabreichung.

Auch die erfindungsgemäßen Polynucleotide können zur Behandlung von pan-Zytopenien und pan-Zytopathien verwendet werden. Ziel ist dabei die Expression eines PRV-1-Polypeptids oder eines funktionellen Fragments davon in Zellen des betroffenen Patienten. Dabei kommen vor allem Verfahren der Gentherapie zur Anwendung. Zellen des Patienten können isoliert werden und mit einem erfindungsgemäßen Polynucleotid transfiziert werden (ex vivo-Manipulation), um dann dem Patienten wieder zugeführt zu werden. Es sind auch Verfahren denkbar, bei denen die erfindungsgemäßen Polynucleotide durch viralen Transfer in die Zielzellen gelangen. Expression der eingeführten Nucleinsäuren führt dann zu hämatopoietischer Aktivität.

Zur Erläuterung werden folgende Beispiele angegeben.

### Beispiel 1

### Charakterisierung des PRV-Gens

Die folgenden Experimente wurden durchgeführt, um das Gen zu charakterisieren:
- Granulozyten wurden aus Blutkonserven oder Aderlässen von P. vera Patienten nach folgendem Protokoll isoliert:

- Blut wurde mit einem gleichem Volumen an 3% Dextran-Lösung in 0.9% NaCl versetzt und 20 Minuten bei Raumtemperatur (RT) stehengelassen.
- Der Ansatz trennte sich in zwei Phasen. Die obere helle Phase wurde abgenommen und 10 Minuten bei 1800g und RT zentrifugiert.
- Der Überstand wurde verworfen und das Zellpellet im gleichen Volumen 0.9% NaCl resuspendiert.
- Jeweils 35 ml der Zellen in NaCl wurden auf 15 ml Ficoll-Hypaque geschichtet.
- Dann wurde 60 Minuten bei 1800g und RT ohne Bremse zentrifugiert.
- Es bildeten sich ein Zellpellet und zwei Schichten mit einer Interphase.
- Schichten und Interphase wurden abgesaugt und das Zellpellet 30 Sekunden lang in 10 ml eiskalter 0.2% NaCl resuspendiert und nach 30 Sekunden wurden sofort 10 ml eiskalte 1.6% NaCl zugegeben.
- Die Zellen wurden 10 Minuten bei 1800g und RT abzentrifugiert.
- Dann wurde einmal in 10 ml PBS gewaschen und abzentrifugiert.
- Das Zellpellet enthielt 95-99% reine Granulozyten.
- Aus diesen Zellen wurde mit Standardmethoden RNA isoliert.
- 10 mg dieser RNA wurden in einem Northern-Blot auf die Expression von PRV-1 untersucht. Als Sonde wurde die gesamte cDNA-Sequenz von SEQ ID Nr. 1 verwendet.

Dieser Versuch wurde an 19 P. vera Patienten und 21 Kontroll-Blutkonserven durchgeführt. Es wurde eine starke Hybridisierung der PRV-1 Sonde bei P. vera Patienten gefunden. Bei gesunden Kontrollen wurde keine Hybridisierung beobachtet.

### Beispiel 2

### PRV-1 hat Wachstumsfaktor-Aktivität

Aus einer schwangeren Maus wurden Embryos am Tage 13,5 nach Befruchtung entnommen. Die fötalen Lebern wurden entnommen. Die darin enthaltenen Zellen wurden mittels Antikörper angefärbt und durch Säulen-Chromatographie für bestimmte Zellen angereichert, für andere Zellsorten depletiert. Es resultiert ein Zellgemisch, das für bestimmte hämatopoietische Vorläuferzellen (sog. colony forming units-erythroid, CFU-E) angereichert ist. So sind in der fötalen Leber insgesamt ungefähr 2% CFU-E, in den angereicherten Zellen aber 30-40% CFU-E.

Diese CFU-E wurden retroviral transfiziert. Dazu wurde 48 Stunden vorher eine sogenannte "packaging cell line", genannt 293-T, ihrerseits transfiziert. 293-T-Zellen sind eine etablierte humane embryonale Nieren-Zellinie. 293-T-Zellen sind stabil mit mehreren Genen eines Retrovirus transfiziert. Werden nun diese 293-T-Zellen mit zwei Plasmiden, genannt pOS und pKAT, transfiziert, produzieren die 293-T-Zellen ein Retrovirus, das murine fötale Leberzellen infizieren kann. Transfiziert man die 293-T-Zellen mit einem leeren pOS-Vektor und pKAT, wird ein "wild typ" Retrovirus produziert, das nur retrovirale Proteine exprimiert. Hat man hingegen in den pOS-Vektor ein humanes Gen einkloniert, z.B. PRV-1, wird ein Retrovirus produziert, das, wenn es Zellen infiziert hat, dieses Protein exprimiert. Das Retrovirus wird von den 293-T-Zellen in das Zellkultur-Medium sezerniert.

Nach zwei Tagen wird das Zellkultur-Medium der transfizierten 293-T-Zellen, welches das Retrovirus enthält, geerntet und einmal durch einen 0,45 µm Filter filtriert. Um die fötalen Leberzellen zu transfizieren, werden diese Zellen mit dem filtrierten Zellkultur-Medium, welches das Retrovirus enthält, vermischt und 2 Stunden bei 1800 rpm, 20°C unter Zugabe von Polybren zentrifugiert. Anschließend werden die transfizierten fötalen Leberzellen in einem Medium kultiviert (Methocult, der Firma Cell Systems), welches zusätzlich zu den üblichen Salzen und Aminosäuren, fötales Kälberserum, 0,0001-0,4 IU/ml Erythropoetin (EPO) und Methylcellulose (0,8%) enthält. Das EPO benötigen die CFU-E, um hämatopoetische Kolonien auszubilden. Durch die Methylcellulose wird das Medium Geleeartig fest, und es gelingt, einzelne Zellen in diesem Gelee zu fixieren, so daß sie sich, anders als in flüssigem Medium, nicht bewegen können. Daher kann beobachtet werden, ob sich aus einer einzelnen Zelle eine hämatopoetische Kolonie formt oder nicht. CFU-Es bilden erythroide Kolonien, also Kolonien, die rote Blutzellen und deren Vorläuferzellen enthalten.

Nach drei Tagen wird gezählt, wie viele hämatopoietische Kolonien sich entwickelt haben. Verschiedene Ansätze werden verglichen. Nicht in jedem Experiment wurden alle Ansätze überprüft; Ansätze 1-3 sind sehr ähnliche Kontrollen und können jeweils einzeln mit Ansatz 4 verglichen werden.
- Ansatz 1:: Zellen, die nicht retroviral transfiziert wurden;
- Ansatz 2:: Zellen, die mit einem leeren pOS-Vektor transfiziert wurden;
- Ansatz 3:: Zellen, die mit einem "green fluorescent Protein" (GFP), einem nicht hämatopoietisch aktiven Protein, transfiziert wurden.
- Ansatz 4:: Zellen, die mit pOS-PRV-1 (Vektor + erfindungsgemäßes Gen) transfiziert wurden.

**Tabelle 1: Die Ergebnisse von drei wie beschrieben durchgeführten Versuchen sind aufgeführt. Die Zahlen geben jeweils die Anzahl der Kolonien an.**

| | **Ansatz 1** | **Ansatz 2** | **Ansatz 3** | **Ansatz 4** |
|---|---|---|---|---|
| | nicht transfiziert | leerer Vektor (pOS) | GFP (pOS-GFP) | PRV-1 (pOS-PRV-1) |
| **Versuch 1** | 116 | 156 | 80 | 326 |
| **Versuch 2** | | 271 | 273 | 410 |
| **Versuch 3** | 120 | | 131 | 291 |

Die Versuche zeigen, daß CFU-E, die mit PRV-1 transfiziert waren, sehr viel mehr Kolonien (bis zu 3-fach erhöht) bilden, als die diversen Kontroll-CFU-E. Dieses Ergebnis deutet darauf hin, daß PRV-1 einen Wachstumsfaktor für CFU-E darstellt.

### Beispiel 3

### Löslichkeit des Wachstumsfaktors PRV-1

Um zu untersuchen, ob PRV-1 einen löslichen Wachstumsfaktor darstellt, oder Zell-Zell-Kontakt notwendig ist, wurde ein weiteres Experiment durchgeführt. Die "Verpackungs-Zellinie", 293-T, produziert nicht nur nach Transfektion mit den pOS- und pKAT-Vektoren ein Retrovirus. Zusätzlich synthetisieren die 293-T-Zellen auch das von dem in pOS klonierten Gen kodierte Protein, also im vorliegenden Fall PRV-1. Ist das Genprodukt ein lösliches Protein, wird es in das Medium, das die "Verpackungs-Zellinie", 293-T, umgibt, sezerniert. Transfiziert man die 293-T-Zellen nur mit dem pOS-Vektor, ohne pKAT, entstehen keine Retroviren. Das Zellkultur-Medium enthält dann nur das lösliche, von den Zellen produzierte Protein. Es wird Medium von pOS-PRV-1-transfizierten Zellen, ohne Retrovirus, mit CFU-Es vermischt, und im Methylcellulose-Medium ausplattiert und die entstandenen Kolonien gezählt.

Folgende Ergebnisse wurden erzielt:

**Tabelle 2: Löslichkeit von PRV-1. Die Zahlen geben jeweils die Anzahl der Kolonien an.**

| | **Ansatz 1** | **Ansatz 2** | **Ansatz 3** | **Ansatz 4** |
|---|---|---|---|---|
| | nicht transfiziert | leerer Vektor (pOS) | GFP (pOS-GFP) | PRV-1 (pOS-PRV-1) |
| **Versuch 4** | | 137 | 187 | 557 |

Auch in diesem Versuch haben CFU-E, die mit PRV-1-haltigem Medium versetzt waren, sehr viel mehr hämatopoietische Kolonien ausgebildet, als Kontroll-Zellen. Aus diesem Ergebnis kann geschlossen werden, daß PRV-1 einen löslichen Wachstumsfaktor darstellt.

### Beispiel 4

Der Wachstumsfaktor PRV-1 ist N-glycosyliert

Aus einer Patientin mit P. vera wurden Granulozyten isoliert und aus diesen Zellen mittels Standardprotokoll Proteinextrakte angefertigt. Diese Proteinextrakte wurden nach dem Protokoll des "N-Glycosidase F Deglycosylation Kits" der Firma Boehringer Mannheim behandelt. Im einzelnen bedeutet dies, daß die Proteinextrakte mit einem "Denaturierungs Puffer" versetzt wurden, 3 Minuten bei 95°C erhitzt wurden und dann entweder nur mit "Reaktions Puffer" oder mit "Reaktions Puffer" plus N-Glycosidase versetzt wurden. Der Ansatz wurde über Nacht bei 37°C inkubiert und die Proteine auf einer PAGE-Gel Elektrophorese mit anschließendem Western Blot analysiert. Das PRV-1 Protein wurde mit einem Antikörper gegen ein Protein mit der Aminosäuresequenz ID-No. 5 detektiert. Die Ergebnisse zeigen, daß aus Granulozyten gereinigtes PRV-1 Protein 60-65 kDa groß ist, während es nach N-Glycosidase-Verdau nur noch 40 kDa groß ist. Dies beweist eindeutig, daß PRV-1 an Asparagin-Resten (Asparagin = N) glycosyliert ist.

## Patentansprüche

1. Verfahren zum Nachweis von Polycythaemia vera, **dadurch gekennzeichnet, daß** man ein PRV-1-Polypeptid ausgewählt aus der Gruppe bestehend aus einem N-glycosylierten Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2;
oder ein Fragment davon mit wenigstens 50 Aminosäuren oder ein
Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2 oder ein
im wesentlichen reines Polypeptid der Sequenz ID Nr. 2;
mit einem oder mehreren gegen das PRV-1-Polypeptid oder ein Epitop davon gerichteten Antikörper in einem Immunoassay umsetzt.

2. Verfahren nach Anspruch 1, **dadurch gekennzeichnet, daß** man als Antikörper einen polyklonalen oder monoklonalen Antikörper gegen das PRV-1-Polypeptid verwendet.

3. Verfahren zum Nachweis von Polycythaemia vera, **dadurch gekennzeichnet, daß** man das FRV-1-Poiynucieotid mit einem RT-PCR-Verfahren oder einem Blotting-Verfahren nachweist.

4. Verwendung von gegen PRV-1 gerichteten polyklonalen oder monoklonalen Antikörpern sowie üblichen Trägern zur Herstellung eines Arzneimittels zur Behandlung von Polycythaemia vera.

5. Verwendung eines Polypeptids ausgewählt aus der Gruppe bestehend aus einem N-glycosylierten Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2;
oder ein Fragment davon mit wenigstens 50 Aminosäuren oder ein
Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2 oder ein
im wesentlichen reines Polypeptid der Sequenz ID Nr. 2;
als Wachstumsfaktor ex vivo.

6. Verwendung eines Polypeptids ausgewählt aus der Gruppe bestehend aus einem N-glycosylierten Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2;
oder ein Fragment davon mit wenigstens 50 Aminosäuren oder ein
Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2 oder ein
im wesentlichen reines Polypeptid der Sequenz ID Nr. 2;
zur Herstellung eines Arzneimittels zur Behandlung von pan-Zytopenien und pan-Zytopathien im Knochenmark und in der Zirkulation.

7. Verwendung eines Polypeptids ausgewählt aus der Gruppe bestehend aus einem N-glycosylierten Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2;
oder ein Fragment davon mit wenigstens 50 Aminosäuren oder ein
Polypeptid umfassend im wesentlichen eine der folgenden Aminosäuresequenzen:
Aminosäuren 1-437 von Sequenz Nr. 2;
Aminosäuren 1-409 von Sequenz Nr. 2;
Aminosäuren 22-437 von Sequenz Nr. 2;
Aminosäuren 22-409 von Sequenz Nr. 2 oder ein
im wesentlichen reines Polypeptid der Sequenz ID Nr. 2;
zur Behandlung von Polycythaemia und/oder Vermehrung körpereigener Zellen und/oder etablierter Zellinien ex vivo oder in vitro.

8. Verwendung eines Polynucleotids umfassend im wesentlichen eine der folgenden Nucleotidsequenzen:
Nucleotide 1-1600 von Sequenz Nr. 1;
Nucleotide 36-1346 von Sequenz Nr. 1;
Nucleotide 36-1262 von Sequenz Nr. 1;
Nucleotide 39-1346 von Sequenz Nr. 1;
Nucleotide 39-1262 von Sequenz Nr. 1;
Nucleotide 99-1346 von Sequenz Nr. 1;
Nucleotide 99-1262 von Sequenz Nr. 1;
zur Herstellung eines Arzneimittels zur Behandlung von pan-Zytopenien und pan-Zytopathien im Knochenmark und in der Zirkulation.

## Claims

1. Process for detecting polycythaemia vera, **characterized in that** a PRV-1 polypeptide selected from the group consisting of an N-glycosylated polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2;
or a fragment thereof containing at least 50 amino acids or a
polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2 or an
essentially pure polypeptide of the sequence ID No. 2;
is reacted, in an immunoassay, with one or more antibodies which is/are directed against the PRV-1 polypeptide or an epitope thereof.

2. Process according to claim 1, **characterized in that** the antibody employed is a polyclonal or monoclonal antibody against the PRV-1 polypeptide.

3. Process for detecting polycythaemia vera, **characterized in that** the PRV-1 polynucleotide is detected using an RT-PCR method or a blotting method.

4. Use of polyclonal or monoclonal antibodies which are directed against PRV-1 as well as customary excipients for producing a drug for treating polycythaemia vera.

5. Use of a polypeptide selected from the group consisting of an N-glycosylated polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2;
or a fragment thereof containing at least 50 amino acids or a
polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2 or an
essentially pure polypeptide of the sequence ID No. 2;
as a growth factor ex vivo.

6. Use of a polypeptide selected from the group consisting of an N-glycosylated polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2;
or a fragment thereof containing at least 50 amino acids or a
polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2 or an
essentially pure polypeptide of the sequence ID No. 2;
for producing a drug for treating pancytopenias and pancytopathies in the bone marrow and in the circulation.

7. Use of a polypeptide selected from the group consisting of an N-glycosylated polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2;
or a fragment thereof containing at least 50 amino acids or a
polypeptide, essentially comprising one of the following amino acid sequences:
amino acids 1-437 of sequence No. 2;
amino acids 1-409 of sequence No. 2;
amino acids 22-437 of sequence No. 2;
amino acids 22-409 of sequence No. 2 or an
essentially pure polypeptide of the sequence ID No. 2;
for treating polycythaemia vera and/or multiplying endogenous cells and/or established cell lines ex vivo or in vitro.

8. Use of a polynucleotide, essentially comprising one of the following nucleotide sequences:
nucleotides 1-1600 of sequence No. 1;
nucleotides 36-1346 of sequence No. 1;
nucleotides 36-1262 of sequence No. 1;
nucleotides 39-1346 of sequence No. 1;
nucleotides 39-1262 of sequence No. 1;
nucleotides 99-1346 of sequence No. 1;
nucleotides 99-1262 of sequence No. 1;
for producing a drug for treating pancytopenias and pancytopathies in the bone marrow and in the circulation.

## Revendications

1. Procédé de mise en évidence de la polyglobulie primitive, **caractérisé en ce que** l'on fait réagir dans un essai immunologique un polypeptide de PRV-1 sélectionné dans le groupe constitué d'un polypeptide N-glycosylé comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou un fragment de celui-ci avec au moins 50 acides aminés, ou
un polypeptide comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou encore un polypeptide sensiblement pur de séquence d'ID N° 2 ; avec un ou plusieurs anticorps dirigés contre le polypeptide de PRV-1 ou l'un de ses épitopes.

2. Procédé selon la revendication 1, **caractérisé en ce que** l'on utilise en tant qu'anticorps un anticorps polyclonal ou monoclonal dirigé contre le polypeptide de PRV-1.

3. Procédé de mise en évidence de la polyglobulie primitive, **caractérisé en ce que** l'on met en évidence le polynucléotide de PRV-1 à l'aide d'un procédé utilisant la RT-PCR ou d'un procédé de transfert.

4. Utilisation d'anticorps polyclonaux ou monclonaux dirigés contre PRV-1, et de supports habituels pour la fabrication d'un médicament destiné au traitement de la polyglobulie primitive.

5. Utilisation en tant que facteur de croissance *ex vivo,* d'un polypeptide sélectionné dans le groupe constitué d'un polypeptide N-glycosylé comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou d'un fragment de celui-ci avec au moins 50 acides aminés, ou d'un polypeptide comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou encore d'un polypeptide sensiblement pur de séquence d'ID N° 2.

6. Utilisation d'un polypeptide sélectionné dans le groupe constitué d'un polypeptide N-glycosylé comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou d'un fragment de celui-ci avec au moins 50 acides aminés, ou d'un polypeptide comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou encore d'un polypeptide sensiblement pur de séquence d'ID N° 2 ; pour la fabrication d'un médicament destiné au traitement de pancytopénies et de pancytopathies dans la moelle osseuse et la circulation.

7. Utilisation d'un polypeptide sélectionné dans le groupe constitué d'un polypeptide N-glycosylé comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou d'un fragment de celui-ci avec au moins 50 acides aminés, ou
d'un polypeptide comprenant pour l'essentiel l'une des séquences d'acides aminés suivantes :
- acides aminés 1 à 437 de la séquence N° 2 ;
- acides aminés 1 à 409 de la séquence N° 2 ;
- acides aminés 22 à 437 de la séquence N° 2 ;
- acides aminés 22 à 409 de la séquence N° 2 ;
ou encore d'un polypeptide sensiblement pur de séquence d'ID N° 2 ; pour le traitement de la polyglobulie primitive et/ou la multiplication de cellules endogènes et/ou de lignées cellulaires établies *ex vivo* ou *in vitro.*

8. Utilisation d'un polynucléotide comprenant pour l'essentiel l'une des séquences de nucléotides suivantes :
- nucléotides 1 à 1600 de la séquence N° 1 ;
- nucléotides 36 à 1346 de la séquence N° 1 ;
- nucléotides 36 à 1262 de la séquence N° 1 ;
- nucléotides 39 à 1346 de la séquence N° 1 ;
- nucléotides 39 à 1262 de la séquence N° 1 ;
- nucléotides 99 à 1346 de la séquence N° 1 ;
- nucléotides 99 à 1262 de la séquence N° 1 ;
pour la fabrication d'un médicament destiné au traitement de pancytopénies et de pancytopathies dans la moelle osseuse et la circulation.
